# EUROPEAN PATENT APPLICATION

(11) **EP 4 487 832 A1**
(43) Date of publication of application: **08.01.2025**
(21) Application number: 23215366.8
(22) Date of filing: 08.12.2023
(51) Int. Cl.: A61J 7/04

(54) **SYSTEM AND METHOD FOR DISPENSING**

(30) Priority: 03.07.2023 US 202318217970
(71) Applicant: Willett, Bradley Paul, Scottsdale, AZ 85251 (US)
(72) Inventor: Willett, Bradley Paul, Scottsdale, AZ 85251 (US)
(74) Representative: Cameron Intellectual Property Ltd

(57) **Abstract**

The present invention is an apparatus and method for a low-cost dispenser designed to control access to substances, including prescribed drugs. When used in conjunction with a prescribed drug, the dispenser controls access to the prescribed drug so that the patient only has access to the prescribed amount of drug at the prescribed frequency and for the prescribed period. The dispenser incorporates a dispenser housing, a rotatable insert, an insert locking system, and a processing system. The insert is rotatable and has compartments that are accessible through a slot in the dispenser housing. Two time periods are set through the processing system and control the insert rotation. The first time period sets the frequency, and the second time period sets the total time over which the frequency may occur.

## Description

### BACKGROUND

The field of the present invention relates to the timed and controlled dispensing of substances.

In reference to a particular indication, certain drugs with bonafide medicinal purposes, such as opioids, can be highly addictive and may be abused. Of the four billion prescriptions given in the United States in 2022, 181 million were for opioids. Six percent of all American adults abuse opioid prescriptions annually, and two million are addicted. On average, 115 Americans die of opioid overdose every day.

In 2022 there were 3.7 million first-time opioid abusers in the United States alone.

Forty percent of all abusers will get their opioids from family and friends. This includes teens, and sixty-two percent of teen abusers are estimated to get their medication simply by sneaking from their family's medicine cabinet.

A prescription, including but not limited to an opioid prescription, incorporates a drug, a dosage, a frequency, and an overall time length. There is yet no effective, low-cost, practical solution to the opioid crisis which accounts for and controls access to each element of the prescription. As such, the opioid crisis cost the United States, for example, 696 billion dollars in 2018 and the lives of thousands of people. In fact, opioid-related overdoses have risen over three hundred percent from 2010 to 2020, from 21,088 per year to 68,630 per year respectively.

### BRIEF SUMMARY OF THE PRESENT INVENTION

The field of the present invention relates to timed and controlled dispensing and compliance thereof. Without limiting the indication or substance for dispensing, the present invention provides mechanisms and methods for metering access to the drug, medicine, nutraceuticals, pet food, and the like, wherein the dosage of a particular regiment or amount is set by a third party (i.e., pharmacist, doctor, vet, etc.) and compliance is beneficial, desired, or required for the safety of the consumer.

In one exemplary embodiment, the present invention provides an apparatus and method for dispensing any substance, wherein the dosage of a particular regiment or amount is set by a third-party (e.g., pharmacist, doctor, vet, etc.), and compliance is required for the safety of the consumer.

In at least one exemplary embodiment, the dispenser comprises a housing having an inner surface and an outer surface, the inner surface defining a housing inner volume, the housing having an opening formed therein that extends between the inner and outer surfaces; an insert rotationally mounted within the housing inner volume, the insert comprising a plurality of compartments along a proximal edge thereof, such that only one of the compartments at a time is accessible via the opening, and a different one of the compartments is accessible via the opening each time the insert is rotated; an insert lock disposed within the housing inner volume and selectively movable between a lock position, in which the insert lock prevents rotation of the insert, and an unlock position, in which the insert lock does not prevent rotation of the insert; and a processing system disposed within the housing inner volume and in operable communication with the insert lock, the processing system configured to (i) move the insert lock from the lock position to the unlock position at a first predetermined time, (ii) move the insert lock from the unlock position to the lock position each time the insert is rotated the predetermined amount, and (iii) prevent the insert lock from being moved from the lock position to the unlock position after a second predetermined time.

For clarification, and in at least one exemplary embodiment, the dispenser would be filled with a particular regiment or amount by a third party (i.e., pharmacist, doctor, vet, etc.). This third party would then program the dispenser with the first predetermined time for dispensing of an allotment or dose in the first compartment. It is contemplated within this invention that the first predetermined time may be a date and time or may be a set time period after the third-party transfers the dispenser to the consumer. For purposes of this invention, a consumer or user may be the actual consumer of the dispensed substance or the custodian/caregiver of the person or animal that actually consumes the dispensed substance.

Further, in at least one exemplary embodiment, the third-party would additionally program the dispenser with the second predetermined time, thereby limiting the allotment or dose in subsequent compartments to be dispensed across two (2) time constraints. For example, a pharmacist could prescribe an antibiotic to be dispensed every four (4) hours but no more than three (3) pills in one day. The first and second predetermined times would allow for this in at least one exemplary embodiment.

In relation to a prescription, the first-time setting would control the frequency of a prescription and the second time setting would control the prescription length. The second time setting would prevent access to the medication after the prescription is completed, and the first time setting would control how often the user can take a medication dose.

In at least one exemplary embodiment, a display screen is disposed on the outer surface of the housing, operably connected to the processing system. This display screen may operate to display, but is not limited to, displaying system information, information related to dosing, and allow a third-party to program the dispenser with the first and second predetermined times.

In at least one exemplary embodiment, there is a second insert lock disposed on the outer surface of the housing and selectively movable between a lock position, in which the second insert lock prevents rotation of the insert, and an unlock position, in which the second insert lock does not prevent rotation of the insert.

In at least one exemplary embodiment, the insert is configured to act as a rachet, and a pawl, disposed within the housing volume and operably connected to the insert, is configured to limit the insert rotation. The rachet and pawl system may limit the rotational direction of the insert to one rotational direction.

In at least one exemplary embodiment, the processing system is configured on a circuit comprising a processor, a clock, a controller, a battery, and a memory housed on a printed circuit board operationally disposed in the inner volume of the housing.

In at least one exemplary embodiment, there is a housing lock system (i) disposed within the housing volume and accessible on the outer surface of the housing and (ii) selectively configurable between a locked state, in which the interior housing volume is inaccessible to a user, and an unlocked state in which the interior housing volume is accessible to the user.

In at least one exemplary embodiment, there is an emergency compartment disposed within the housing volume and accessible on the outer surface of the housing. This emergency compartment may be lockable. In at least one embodiment the emergency compartment is of suitable dimension to contain a dose of Narcan. This allows the user of the dispenser quick access to Narcan in case of overdose as the Narcan and the dispensed substance do not need to be carried separately but may be housed in the same dispenser.

In one exemplary embodiment, the present invention provides an apparatus and method for a low-cost dispenser designed to control access to drugs. The dispenser is suitable for controlling prescribed drugs or substances that have not been prescribed. When used in conjunction with a prescribed drug, the dispenser controls access to the prescribed drug so that the patient only has access to the prescribed amount of drug at the prescribed frequency and for the prescribed period. The dispenser may be portable and tamper-resistant.

In one exemplary embodiment, the dispenser may be used in conjunction with opiates, other controlled substances by the Drug Enforcement Administration, or other drugs.

In at least one exemplary embodiment, there is a child-proof emergency dose capsule disposed in the housing's inner volume and accessible on the outer surface of the housing.

In at least one exemplary embodiment, the housing lock system comprises a tamper-evident screw system.

In at least one exemplary embodiment, the housing lock system comprises a snap lock system.

In at least one exemplary embodiment, each of the plurality of compartments have no underside such that when a compartment is accessible via the opening, the regiment or amount of substance can be dispensed into a secondary container.

In at least one exemplary embodiment, a dose dispensing system is disclosed wherein multiple dispensers are utilized in concert.

In at least one exemplary embodiment, a first and second dispenser, with a plurality of compartments having no underside, the housings having radio frequency identification (RFID) configured to provide information about the substances contained therein, and the programed first and second predetermined times, respectively, and a base unit configured to receive the first and second dispensers comprising (i) an enclosure defined by the first device and second device side walls, thereby allowing the openings of the first and second dispensers to align; (ii) a processing system configured on a printed circuit board operationally disposed of in a base repository, further comprising sensors to identify the first and second dispensers; and (ii) a dispensing area or secondary container for collecting the substances contained within the aligned compartments of the first and second dispensers.

For example, pharmacist A could prescribe an opioid and program Dispenser One (1) to be dispensed every four (4) hours, but no more than three (3) pills in one day, and pharmacist B could prescribe an antibiotic and program Dispenser Two (2) to be dispensed every eight (8) hours, but no more than two (2) pills in one day. Both Dispenser One (1) and Dispenser Two (2) would be placed in a base unit, and dispense both the opioid and the antibiotic into the dispensing area or secondary container for the consumer.

It is contemplated within the scope of this invention that the multiple dispensers may be stacked into one base unit to allow for fast and efficient delivery of dosing in a commercial setting (i.e., nursing home) to relieve labor and increase compliance.

It is also contemplated within the scope of this invention that the base unit may have additional logic such that the programmed predetermined times from each dispenser may be consolidated around a more convenient 24-hour consumer schedule.

In at least one exemplary embodiment, the method of providing a substance to a user comprises: Providing a dispenser to the user comprising: a housing having an inner surface and an outer surface, the inner surface defining a housing inner volume, the housing having an opening formed therein that extends between the inner and outer surfaces; an insert rotationally mounted within the housing inner volume and rotatable relative to the housing, the insert comprising a plurality of compartments along a proximal edge thereof, such that only one of the compartments at a time is accessible via the opening, and a different one of the compartments is accessible via the opening each time the insert is rotated a predetermined amount; an insert lock disposed within the housing inner volume and selectively movable between a lock position, in which the insert lock prevents rotation of the insert, and an unlock position, in which the insert lock does not prevent rotation of the insert; and a processing system disposed within the housing inner volume and in operable communication with the insert lock, the processing system configured to (i) move the insert lock from the lock position to the unlock position at a first predetermined time, (ii) move the insert lock from the unlock position to the lock position each time the insert is rotated the predetermined amount, and (iii) prevent the insert lock from being moved from the lock position to the unlock position after a second predetermined time period; Providing to the user a series of doses, each of the doses operably enclosed by at least one of the plurality of compartments.

In at least one exemplary embodiment, the first predetermined time period of the provided dispenser increases in length of time by a fixed amount of additional time after the predetermined event. For example, the dispenser may be used to dispense an opioid to a user. The dispenser may be programmed to increase the time period after the user takes a drug. This allows the user to taper off opioid use.

In at least one exemplary embodiment the provided dispenser, further comprises a second insert lock disposed on the outer surface of the housing and selectively movable between a lock position, in which the second insert lock prevents rotation of the insert, and an unlock position, in which the second insert lock does not prevent rotation of the insert.

In at least one exemplary embodiment, the provided dispenser further comprises, a child-proof emergency dose capsule disposed in the housing inner volume and accessible on the outer surface of the housing.

In at least one exemplary embodiment, the provided dispenser further comprises, a display screen disposed on the outer surface of the housing and operably connected to the processing system. This display screen may display information regarding the dispenser and the substance it contains. However, the display screen may display anything it is programmed to display.

In at least one exemplary embodiment, the insert of the provided dispenser is configured to act as a rachet, and a pawl, disposed within the housing volume and operably connected to the insert, is configured to limit the insert to rotation in the first rotational direction.

In at least one exemplary embodiment, the processing system of the provided dispenser is configured on a circuit comprising a processor, a clock, a controller, a battery, and a memory housed on a printed circuit board operably disposed in the inner volume of the housing.

In at least one exemplary embodiment, the provided dispenser further comprises a housing lock system (i) disposed within the housing volume and accessible on the outer surface of the housing and (ii) selectively configurable between a locked state, in which the interior housing volume is inaccessible to a user, and an unlocked state in which the interior housing volume is accessible to the user.

In at least one exemplary embodiment, the housing lock system of the provided dispenser comprises a tamper-evident screw system. In at least one exemplary embodiment, the housing lock system of the provided dispenser comprises a snap lock system.

In at least one exemplary embodiment, the provided device may have an insert with compartments, and each of the plurality of compartments having no underside such that when a compartment is accessible via the opening, the regiment or amount of substance can be dispensed into a secondary container. This allows for a dose dispensing system wherein multiple dispensers are utilized in concert.

For example, in at least one exemplary embodiment, a first and second dispensers, as described above with e plurality of compartments have no underside, further comprising RFIDs configured to provide information about the substances contained therein; and the programed first and second predetermined times, respectively, and a base unit configured to receive the first and second dispensers comprising (i) an enclosure defined by the first device and second device side walls, thereby allowing the openings of the first and second dispensers to align; (ii) a processing system configured on a printed circuit board operationally disposed in base repository, further comprising sensors to identify the first and second dispensers; and (ii) a dispensing area or secondary container for collecting the substances contained within the aligned compartments of the first and second dispensers.

### BRIEF DESCRIPTION OF THE FIGURES

FIG. 1 is a perspective view of a dispenser housing with an opening and an insert, the insert aligned so that a compartment of the insert shows in the opening.
FIG. 2 is a three-dimensional cross-section view of a dispenser housing with an opening.
FIG. 3 is a perspective view of a dispenser housing with a through opening.
FIG. 4 is a blown-out view of a dispenser housing where the dispenser housing comprises a top portion and a bottom portion.
FIG. 5 is a top down-perspective view of a rotatable insert of the present invention.
FIG. 6 is a perspective view of an exemplary embodiment of the dispenser.
FIG. 7 is a top-down perspective view of a rachet and pawl system where the insert serves as the rachet.
FIG. 8 is a top-down perspective view of an interior insert lock system where the lock is a solenoid lock and is in an unlocked state.
FIG. 9 is a top-down perspective view of an interior insert lock system where the lock is a solenoid lock and is in a locked state.
FIG. 10 is a diagram showing the initiation and operation of the timing systems initiated by a wake event.
FIG. 11 is a block diagram of the circuit of an exemplary embodiment of the present invention.
FIG. 12 is a sectional view of a coin-turned-emergency dose capsule.
FIG. 13 is a cross-sectional view of a coin-turned emergency dose capsule sitting in the dispenser housing.
FIG. 14 is a perspective view of an external insert locking system in a locked state.
FIG. 15 is a perspective view of an external insert locking system in an unlocked state.
FIG. 16 is a cross-sectional view of a dispenser housing with a screw-based housing lock system.
FIG. 17 is a cross-sectional view of a dispenser housing with a locked snap lock-based housing lock system.
FIG. 18 is a cross-sectional view of a dispenser housing with a snap lock-based housing lock system.
FIG. 19 is a blowout view of an exemplary embodiment of the dispenser.
FIG. 20 is a perspective view a dispenser housing with a removable display.
FIG. 21 is a perspective view of a removable display.
FIG. 22 is a diagram showing a possible flow of the dispenser and the substance from the user and provider.
FIG. 23 is a diagram showing a possible flow of the dispenser and the substance from the providers to a user where the user is presented with the substance the dispenser.
FIG. 24 is a diagram showing a possible flow of the dispenser and the substance from the two providers to a user.
FIG. 25 is a diagram showing a possible alternate flow of the dispenser and the substance from the user and provider where the dispenser is returned by the user.
FIG. 26 is a diagram showing a provider providing a dispenser and a substance to a user, the user providing the dispenser and or substance to a third party, and the third party providing the dispenser back to the provider.
FIG. 27 is a flowchart of a method of the present invention where the pharmacist receives a prescription and provides the fulfilled prescription to the user according to the methods of the present invention.
FIG. 28 is a flowchart of an exemplary method of the present invention showing that a dispenser of the present invention may be filled, programmed, or modified by the pharmacist.

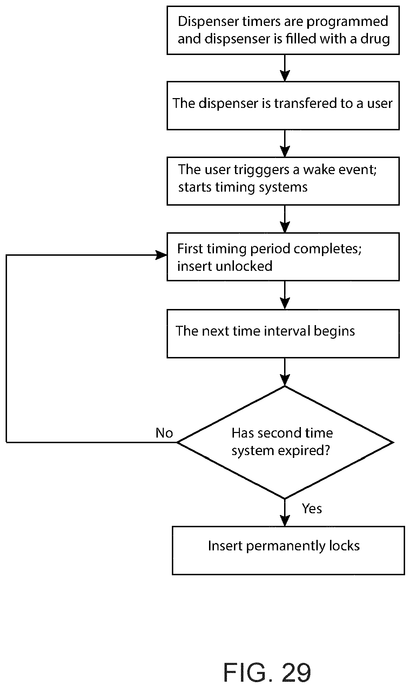

FIG. 29 is a flowchart of an exemplary method of the present invention where the first timing period repeats until the second timing system permanently locks the dispenser insert.

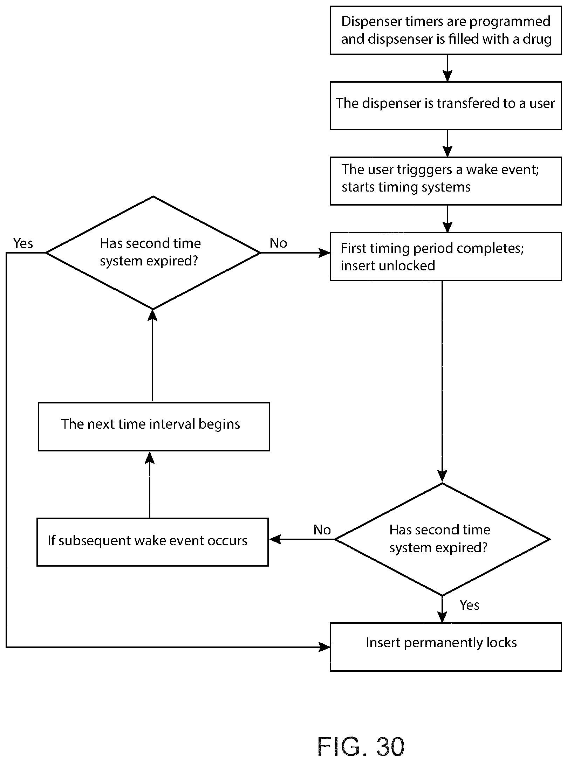

FIG. 30 is a flowchart of an exemplary method of the present invention where, conditioned on the occurrence of a subsequent wake event; the first timing period repeats until the second timing system permanently locks the dispenser insert.
FIG. 31 is a perspective view of a dispenser having a through-opening with a modified insert.
FIG. 32 is a perspective view of a base repository for dispensers, holding two dispensers operably stacked to provide access to multiple medications.

### DETAILED DESCRIPTION OF THE INVENTION

The present invention relates to the field of dispensers. Without limiting the indication or substance for dispensing, the present invention provides mechanisms and methods for metering access to medicine, nutraceuticals, pet food, and the like, wherein the dosage of a particular regiment or amount is set by a third party (i.e., pharmacist, doctor, vet, etc.) and compliance is beneficial for the safety of the consumer.

There is a need to account for multiple variables to properly control a substance's dispensing. For example, when the substance is a drug and is incorporated into a prescription.

In a prescription, a drug is assigned a dosage, a frequency, and an overall time length for a patient who is a user of the prescription. Therefore, for compliance in a medical environment, the dispenser of the present invention is designed to control access to the drug according to the dosage, frequency, and overall time length of the prescription. In light of the opioid crisis a hope of this invention is to prevent access to prescribed drugs beyond the exact parameters of the prescription (e.g., frequency and prescription length). By limiting access according to prescription frequency, the user of the drug is limited to an as needed basis. By limiting access according to prescription length, the user cannot access the drugs after the completion of the prescription.

In at least one exemplary embodiment, the dispenser of the present invention is designed for the pharmacist to be able to fill and give to the patient for home use while reducing the access potential of a user or potential user, such as a friend or relative of the patient or other third party. With a simple pill bottle, access to the medication is unlimited, but with the dispenser of the present invention, access to the medication is limited to the prescription parameters.

In at least one exemplary embodiment, the dispenser, the drugs are filled as part of the manufacturing process. Thus, a pharmacist might not have access to the drugs. Although, a user of the dispenser may have access according to pre-set parameters (e.g., frequency, dose, period) set during dispenser manufacturing. In such cases, a pharmacist may select a prefilled and preprogrammed dispenser to give to the user, who may then wake and use the dispenser.

The dispenser limits access to the substance by incorporating a dual timing system where one timing system controls the frequency of the dispensing and the second time system controls the overall period that the dispenser is active. Thus, in the context of the prescription, it can be said that the first timing system controls the prescription frequency, and the second timing system controls the prescription length. The timing system is such that when the first timer is active or the second timer is inactive, the device is locked; and when the first timer is inactive and the second timer is active, the device is unlocked.

In reference to the dispenser, in at least one exemplary embodiment, the dispenser incorporates a housing, an insert, an insert locking system, and a processing system.

In at least one exemplary embodiment, the dispenser housing has an inner surface and an outer surface, the inner surface defining a housing inner volume, the housing having an opening formed therein that extends between the inner and outer surfaces.

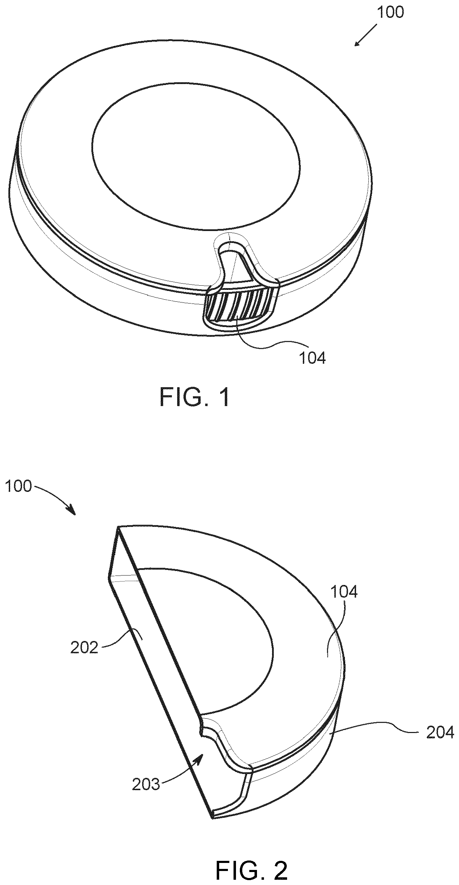

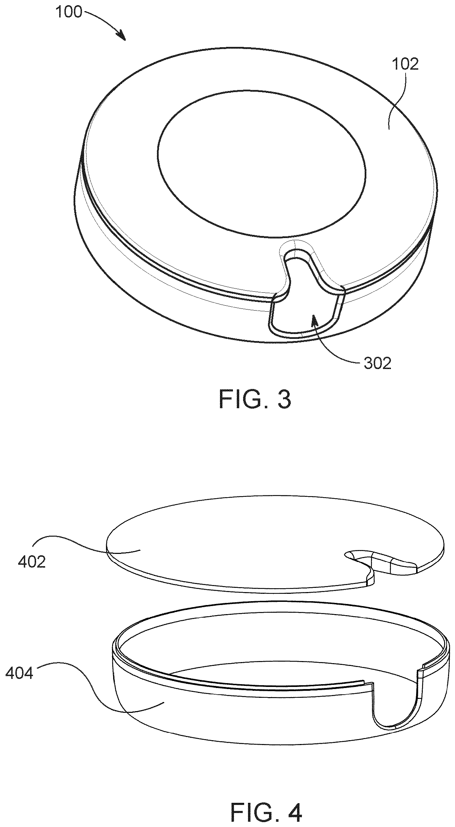

Referring to first to FIG. 1, one embodiment of a dispenser 100 for controllably dispensing a controlled substance, such as prescription medication, is depicted and includes at least a housing 102 and an insert 104. The housing 102, as shown more clearly in FIGS. 2-4, has an inner surface 202 that defines a housing inner volume 203, and an outer surface 204. The housing 102 also has an opening 302 formed therein that extends between the inner and outer surfaces 202, 204. It will be appreciated that in some embodiments, the housing 102 may be configured to allow access to the inner volume 203. As shown in FIG. 4, this accessibility may be implemented, at least in part, by forming the housing 102 from a first portion 402 and a second portion 404, and such that the first portion 402 is at least selectively separable, using techniques described below, from the second portion 404.

Before proceeding further, it is noted that the housing 102 may be constructed of any one of numerous materials. Some non-limiting examples of suitable materials include various metals, metal alloys, plastics, or a combination thereof, just to name a few. To produce an affordable disposable dispenser (a dispenser which is intended to be disposed after completion of a single dispensing regime) it may be preferred to use plastic. Therefore, the suitability of the material for the dispenser may vary according to the intended lifespan of the dispenser.

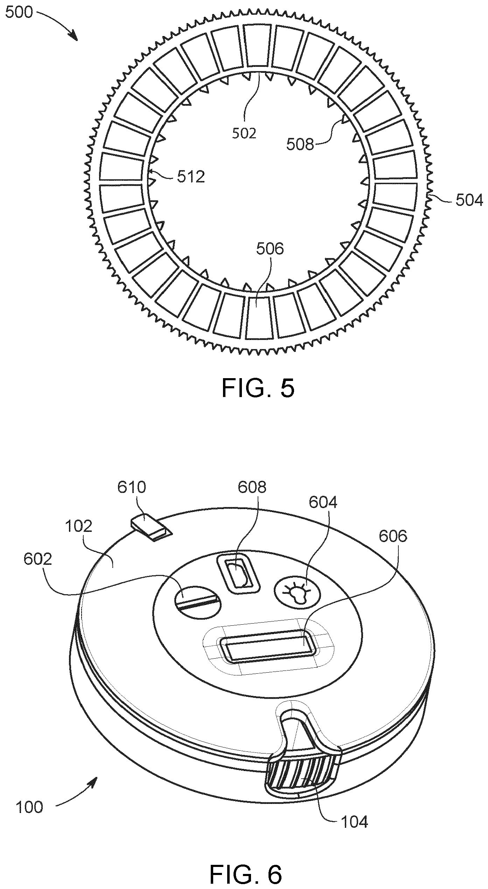

The insert 104 is rotationally mounted within the housing inner volume 203 and is rotatable relative to the housing 102. The insert 104, an embodiment of which is depicted in FIG. 5, includes an inner peripheral surface 502, an outer peripheral surface 504, and a plurality of compartments 506 (e.g., a chain of compartments 506 in the depicted embodiment). The depicted insert 104 has a plurality of pawl catches 508 formed on the inner peripheral surface 502, and a rod catch space 512 is defined between successive ones of the pawl catches 508. The purpose of the pawl catches 508 is described further below. The outer peripheral surface 504 may be configured to be textured, as depicted in the embodiment of FIG. 5, or it may be smooth.

Regardless of whether or not the outer peripheral surface 504 is textured, it is seen that the compartments 506 are disposed along a proximal edge of the insert 104 between the inner and outer peripheral surfaces 502, 504. It will be appreciated that the number of compartments 506 may vary. For example, the depicted insert includes 30 compartments. However, the insert 104 may have any number of compartments, including various odd and even numbers, and as needed or desired for a particular prescription or use.

Compartments 506 of the insert 500 are intended to receive and hold substance, for example, a medication. To facilitate quick loading of medication, the substance may be prepackaged in a blister pack which is shaped as a circle of blisters according to the insert dimensions of the insert that will receive the substance with each blister of the blister pack being shaped and sized to fit into a compartment of the insert.

This blister pack is intended to improve the loading of the insert with substances as well as the longevity of the substance. Because the blister pack is dimensioned according to the insert it may be placed over the insert and pressed down. The pressing process would separate the blisters of the blister package as the insert compartment walls resisted the blister pack causing the blisters to split. The blisters would then fall into the compartments. Keeping a substance in a blister prevents environmental interaction between the substance and the environment, helping to protect the substance from degrading and improving the substance's longevity. The use of a blister pack or other pack is useful in automating the loading of the insert. In at least one exemplary embodiment, when a blister pack is used, the blister itself is dispensed and the user removes the substance from the blister.

As FIG. 1 clearly depicts, the insert 104 and the opening 302 are disposed and dimensioned such that only one of the compartments 506 at a time is accessible via the opening 302. Moreover, and as will be further described, a different one of the compartments 502 is accessible via the opening 302 each time the insert 104 is rotated a predetermined amount.

As with the housing 102, it will be appreciated that the insert 104 may also be constructed of any one of numerous materials. Some non-limiting examples of suitable materials include various metals, metal alloys, plastics, or a combination thereof, just to name a few. The insert 104 may also, in at least some embodiments, be disposable. While the insert 104, at least in the depicted embodiment, is substantially circular, it may, in other embodiments, take on any one of numerous non-circular shapes. In addition, in at least one exemplary embodiment, the housing 102 and/or insert 104 may be dimensioned to have two or more inserts 104 disposed therein.

Before proceeding further, it is noted that the depicted housing 102 is circular in shape. This, at least in part, is so that this shape optimizes the integration of the rotatable insert 104. It will be appreciated, however, that other housing shapes may be used. For example, a triangle-shaped housing may also be used,. For example, and as FIG. 6 depicts, in some embodiments the housing 102 may be implemented with one or more of an emergency dose capsule 60602, a wake switch 60604, a display device 60606, a suitable input/output (I/O) interface 60608, such as a USB port, and/or an external lock system 61610, which may serve as a child proof lock. The housing 102 may also be implemented with various types of non-illustrated housing lock systems, which are configured to prevent access to the housing inner volume 203. Each of these various features and devices will be described further below.

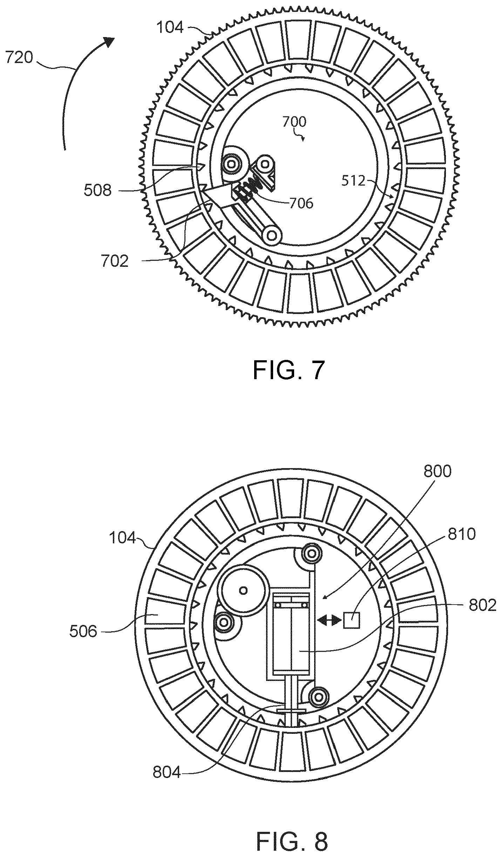

As noted above, the insert 104 is rotatable relative to the housing 102. In at least one exemplary embodiment, the predetermined amount of rotation corresponds to the dimension of one compartment 506. In at least one exemplary embodiment, the insert rotation itself is driven by the application of a force originating outside the dispenser, for example, from a user who applies forces on the insert via his fingers through the opening 604, in such embodiments the insert is referred to as being externally rotated. In at least one exemplary embodiment, and as will be described momentarily, the capability to rotate, and the amount of rotation, is controlled by an insert lock, which is configured to prevent rotation of the insert 104 after a predetermined amount of rotation. Moreover, the rotational direction of the insert 104, in at least one exemplary embodiment, may be limited to a single rotational direction. One configuration of a mechanism for limiting insert rotation to a single direction is depicted in FIG. 7 and will now be described.

In the embodiment depicted in FIG. 8, a ratchet and pawl system 700 is used to limit insert rotation to a single direction. The ratchet and pawl system 700 includes the previously mentioned plurality of pawl catches 508 and a spring-biased pawl 702. The pawl catches 508, which serve as ratchets, are formed, as was noted above, on the inner peripheral surface 502 of the insert 104. The pawl 702 is configured to limit the rotation of the insert 104 to one direction 720. Specifically, when the insert 104 is rotated in the proper, single direction 704, one of the pawl catches/ratchets 508 will push the pawl 702, against the spring force of a spring 706, out of the rod catch space 512 that the pawl 702 is in. The pawl 702 will then drop, due to the spring force, into the next rod catch space 512. As will be described further below, each incremental rotation of the insert 104 may, in some embodiments, trigger a microswitch (not visible in FIG. 7). More specifically, in such embodiments, each time the pawl 702 drops into the next catch space 512, it preferably triggers the microswitch. No matter how the microswitch is triggered, it in turn activates the above-mentioned insert lock, an embodiment of which will now be described.

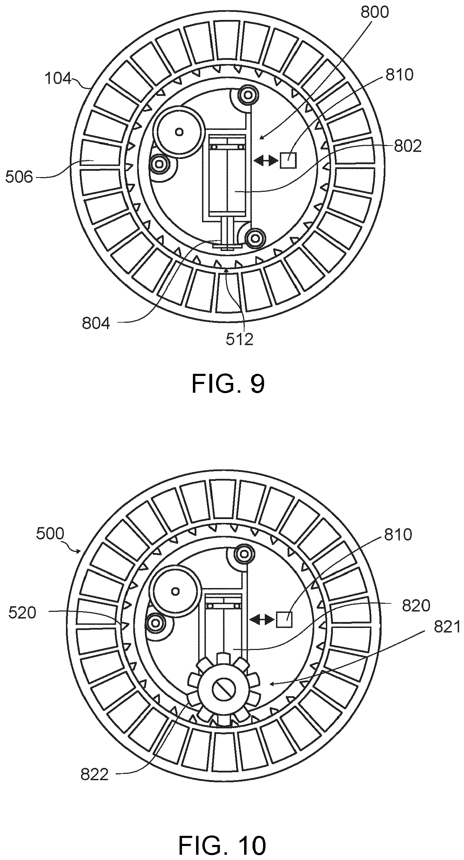

The insert lock 800, an embodiment of which is depicted in FIGS. 8 and 9, is disposed within the housing inner volume 203 and is selectively movable between a lock position, which is the position depicted in FIG. 8, and an unlock position, which is the position depicted in FIG. 9. In the lock position, the insert lock 800 prevents rotation of the insert 104, and in the unlock position, the insert lock 800 does not prevent rotation of the insert 104. It will be appreciated that the insert lock 800 may be variously configured to implement this functionality, in the depicted embodiment, however, the insert lock 800 includes a solenoid 802 and a lock rod 804. The lock rod 804 is coupled to the solenoid 802, and the solenoid 802 is configured, when it is electrically de-energized, to move the lock rod 804 into, or keep the lock rod 804 in, the lock position. As was noted above, the insert 104 has a rod catch space 512 defined between successive ones of the pawl catches 508. Thus, as FIG. 8 depicts, in the lock position, the solenoid 802 moves the lock rod 804 into, or keeps the lock rod 804 in, one of the rod catch spaces 512, thereby preventing rotation of the insert 104 in the predetermined direction. Conversely, and as FIG. 9 depicts, when it is energized, the solenoid 802 moves the lock rod 804 out of, or keeps the lock rod 804 out of, one of the rod catch spaces 512, thereby allowing rotation of the insert 104 in the predetermined direction. Although the solenoid 802 may be variously implemented, in one embodiment, it is implemented using a latching solenoid. It will additionally be appreciated that in other embodiments, a motor may be used to move the lock rod 804, instead of the solenoid 802.

In at least one exemplary embodiment the insert rotation is internally driven and occurs according to a motor assembly which is configured to rotate the insert. This allows the rotation of the insert to be fully controlled by the processing system. The motor may serve in place or in addition to an insert locking mechanism as well as a rachet and pawl system, as the insert will not rotate unless the motor drives the rotation. In at least one exemplary embodiment, where the motor assembly is used as an insert lock, if the motor is actively rotating the insert, the insert is in an unlocked state, and when the motor is not rotating the insert, the insert is in an locked state.

For example, as shown in FIG. 10, in at least one embodiment, the motor 920 is a gear-based motor where the motor 920 contains a motor gear 821 with motor gear teeth 822 and the insert 500 operates as a second gear having a insert gear teeth 520. The motor gear 821 is an external spur gear with outward facing teeth 822 and the insert 500 is an internal spur gear with inward facing teeth 520. In some cases, the insert gear teeth 520 may be the rod catches of inserts of other embodiments so that a single insert 500 may be made which is usable for both a motor driven insert rotation and an externally rotated insert. The teeth 822 of motor gear 821 and the teeth 520 of the insert 500 are operationally sized and spaced so that the motor gear 821 shall turn the insert 500. Thus, the inward teeth 520 of the insert 500 will interlock with the outward teeth 822 of the motor gear 821 which sits within the insert 500. When motor 920 turns the motor gear 821, the teeth 822 of the motor gear 821 rotate the insert 500 by pushing the interlocked insert teeth 520. When motor 920 does not rotate motor gear 821 the insert 500 will not rotate because the teeth 822 of the motor gear are interlocked with the teeth 520 of the insert.

To turn the motor gear 920 the motor 821 may be operationally placed above the motor gear 821 or the motor gear may be placed to the side of the motor gear 821 and operationally connect to the motor gear by way of a bevel gear. FIG. 10 shows motor 920 above the motor gear 821.

The motor is an electric motor, and it drives the rotation of the motor gear which in turns rotates the insert. Because the motor apparatus may act like a locking mechanism, the motor apparatus may take the space that would otherwise be needed for other insert locking mechanisms.

Both internally rotated and externally rotated embodiments of the present invention are controlled by a processing system. For example, as shown in FIG. 8 and 0 with insert locks, the insert lock 800 is controlled by a processing system 810. As FIGS. 8 and 9 also depict, the processing system 810 is preferably disposed within the housing inner volume 203 and is in operable communication with the insert lock 800. This holds true for the internally rotated embodiments where the processing system is operable communication with the motor. With an externally rotated embodiment, the processing system 810 is configured to move the insert lock 800 between the lock and the unlock position, and thereby selectively enable rotation of the insert 104, according to the dosage frequency of a prescribed medication, and the overall length of the prescription. Thus, the dispenser controls access to the prescribed medication according to the variables of the prescription.

More specifically, the processing system 810 is configured to move the insert lock 800 from the lock position to the unlock position a first time period after a predetermined event, and to move the insert lock 800 from the unlock position to the lock position each time the insert 104 is rotated. The processing system 810 is also preferably configured to prevent the insert lock 800 from being moved from the lock position to the unlock position after a second predetermined time period.

To implement this functionality, the processing system 810 is configured to implement what is referred to herein as a first timer and a second timer. As will be described, the first timer period is either uniform in length or it may change in length at least once during the second predetermined timer period.

In at least one exemplary embodiment, the first timer period is set according to the dosage frequency of the prescription; that is, the time between each dose. Thus, the first timer may be referred to herein as the "next dose timer." The next dose timer, as noted above, implements the first time period after a predetermined event. This predetermined event may vary. For example, the predetermined event may be rotation of the insert 104 or it may the movement of the insert lock 800 into the lock position. In at least one exemplary embodiment, when the predetermined event is the rotation of the insert 104, the rotation of the insert 104 will trigger, for example, a microswitch (described below), which will in turn cause the next dosage timer to implement the first time period. In such embodiments, there will preferably be no prescription dose in the first compartment 504 that is accessible via the opening 302. There are some instances for which the dosage frequency of a prescription may vary over the length of the prescription. Thus, for these types of prescriptions, the first time period may also vary. For example, the dosage frequency of some prescriptions may decrease over the length of the entire prescription. Thus, the first time period implemented by the next dose timer may increase after each predetermined event.

The second time period is set to match the length of time for the entire prescription. Thus, the second timer may be referred to herein as the "subscription end timer." After the subscription end timer implements the second time period, the processing system 810 prevents the insert lock 800 from being moved from the lock position to the unlock position. Therefore, the first and second timers do not generate successive time periods relative to each other, rather the first and second time periods run concurrently, with the first timer (e.g., next dose timer) repeatedly implementing the first period until the second timer (e.g., subscription end timer) implements the second time period.

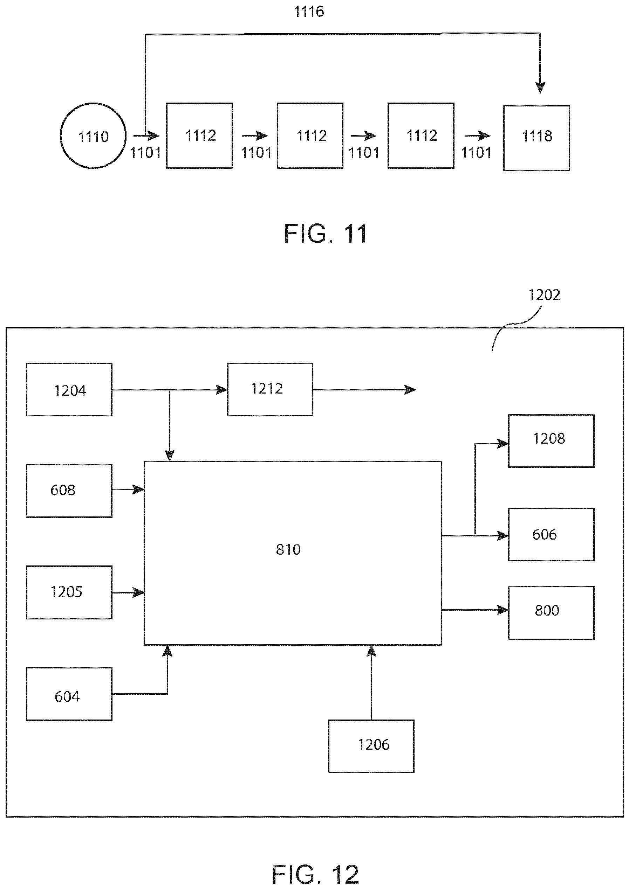

To illustrate the above-described functionality of the first and second timers, reference should now be made to FIG. 11, which depicts a diagram of the first and second timers when initiated by an initiation event 1110. It will be appreciated that the initiation event may vary. For example, in one embodiment the initiation event is a user pressing the previously mentioned wake switch 60604, and in another embodiment the initiation event is an initial rotation of the insert 104. No matter the specific initiation event, after the initiation event 1110, the processing system 810 moves the insert lock 800 to the unlock position to enable a user to rotate the insert 104 the predetermined amount of rotation in the predetermined direction, to thereby allow access to the next compartment 506. In FIG. 11, each successive block 1112 after the initiation event 1110 represents a rotation of the insert 104 to a new compartment 506, each line 1101 represents the first time periods implemented by the first timer (e.g., next dose timer), and line 1116 represents the second time period implemented by the second timer (e.g., subscription end timer). As clearly depicted, after the second time period is complete 1118, the first timer does not continue. As such, at the end of the second time period 1118, regardless of the number of first time periods that have occurred, the insert lock 800 can no longer be moved to the unlock position.

In at least one exemplary embodiment, the processing system 810 may be configured to limit the first timer such that only a certain number of first time periods can occur. This can be achieved by setting the sum of the length of each first time period to less than the length of the second time period-effectively setting a minimum dose limit. The minimum dose limit may be implemented if, for example, the user is required to take the substance from the dispenser 100 on an as-needed basis, but not take a number of doses greater than prescribed. For example, if the user is a patient and has a two-month-long prescription for a pain relief pill, the user is supposed to take a pain relief pill on an as-needed basis and has a limit on how many doses they can take in a moment. In this case, the dose frequency may need to only be limited to six hours, but the prescription length could be two months. In the alternate, the number of pills in the insert 104 could be controlled, and not all compartments of the insert 104 will be filled.

Turning now to FIG. 12, a functional block diagram of one embodiment of the processing system 810 and various other electric components that may be used to implement the functions of the dispenser 100 is depicted and will now be described. In the depicted embodiment, processing system 810 and the various components are mounted on a printed circuit board 1202. However, other mounting techniques may be used. The processing system 810, which may be implemented using one or more suitably programmed devices, such as, for example, a microcontroller, a general-purpose processor, or an application specific processor, is coupled to and receives power from a battery 1204 and, as previously noted, is in operable communication with the insert lock 800. The depicted processing system 810 is also in operable communication with the previously mentioned wake switch 6604, display device 606, and I/O interface 60608. It should be noted, however, that these devices, as mentioned previously, are optional. The depicted processing system 810 may also be in operable communication with the previously mentioned microswitch 1205 and a real-time clock 1206. It will be appreciated that the processing system 810 will have the necessary specs to control each of these components. Nonetheless, if, in a particular embodiment, a component is not connected, then the component need not be accounted for by the processing system 810. Therefore, the processing system 810 specs may vary with embodiments.

It will be appreciated that the battery 1204 may be implemented using any one of numerous types of batteries. In one example embodiment, the battery 1204 may be an alkaline battery, such as an E80 battery, or a lithium-ion battery that supplies, for example, 1.5 VDC and is preferably rechargeable. In the depicted embodiment, the battery 1204 is also connected to at least one boost voltage regulator 1212. The boost voltage regulator 1212, when included, is configured to boost the battery voltage to a higher DC voltage. The boost voltage regulator 1212 is additionally configured to regulate and supply the higher DC voltage to other components, such as the insert lock 800 and, when it is included, the display device 60606. The boost voltage regulator 1212 may, for example, boost the 1.5VDC battery voltage to 3.3VDC. An additional boost voltage regulator 1212 may also be included to boost the 1.5VDC battery voltage to, for example, 5.0 VDC to drive the solenoid 802. One or both of these voltages, however, may vary as needed or desired. In at least one exemplary embodiment, the processing system 810 contains several ADC channels, which allow it to monitor the battery voltage. The processing system 810 can be configured to alert the user, via the display device 60606, of a low battery voltage condition. The processing system 810 may also, in some embodiments, be configured to command the insert lock 800 to move to, or remain in, the lock position when the battery 1204 dies. In some embodiments, the processing system 810 may be configured to command the insert lock 800 to move to, or remain in, the unlock position to allow insert 104 rotation just before or as the battery 1204 dies.

The display device 60606, when included, is in operable communication with, and receives display commands from, the processing system 810. It will be appreciated that the display device 606 may be variously implemented. For example, it may be implemented using a liquid crystal display (LCD) device or an organic light-emitting diode (OLED) display device, just to name two non-limiting examples. In a preferred embodiment, the display device 606 is an OLED display device since the display device allows for greater customization of the display screen as compared to an LCD display device and, unlike the LCD display device, it does not need to be backlit. An OLED display device also consumes less power than an LCD display device. However, an LCD display device is less costly than an OLED screen.

The information that the display may present includes messages to the user, messages to the provider, and messengers to the third party (e.g., the device is a stolen device). The display may be programmed to display messages such as contact information for crisis or help lines. The display may be programmed to display certain messages upon certain events if such a sensor is included in the device to sense such events. For example, upon a forced unlock of the device by physical means, a micro-switch may be triggered, and the device may be programmed to wake and display a message upon the triggering of the micro-switch.

In at least one exemplary embodiment, the processing system 810 is preferably configured for low power operation and only wakes in response to the occurrence of an initiation event 1110. The initiation event 1110, as previously mentioned, may be a rotation of the insert 104 or, when the wake switch 604 is included, a press of the wake switch 604. In those embodiments for which the initiation event 1110 is the rotation of the insert 104, this rotation, as previously noted, triggers the microswitch 1105. The microswitch 1105 is configured, upon being triggered, to supply a lock signal to the processing system 810. The processing system 810, in response to the lock signal, moves the insert lock 800 to the lock position.

In those embodiments for which the initiation event 1110 is the pressing of the wake switch 604, it is configured, when pressed by a user, to supply a wake signal to the processing system 810 for a predetermined time period. The predetermined time period may vary and may be set at any length during manufacturing or during the programming of the processing system 810 via the I/O interface 608 (described below). One example of an optimal time period is about a two-minutes. Regardless of the length of the optimal time period, the wake switch 604, when included, is preferably disposed such that it is readily accessible by a user.

Before proceeding further, it is noted that, at least in some embodiments, the initiation event 1110 may include an initial initiation event 1110 that differs from subsequent initiation event 1110. For example, the initial initiation event 1110 may be the pressing of the wake switch 604, and the subsequent initiation event 1110 may be the rotation of the insert 104. Whether or not the initiation event 1110 is always the same or includes an initial initiation event 1110 that differs from subsequent initiation event 1110, the initiation event 1110, as previously noted, the first and second timers.

Though not previously mentioned, the first and second timers, which are implemented in the processing system 810, are preferably synchronized, with a high degree of accuracy, to real-world time lengths dictated by prescription variables. This is preferably enabled by the real-time clock 1206. It will be appreciated that the real-time clock 1206, when included, may be variously implemented. In at least one exemplary embodiment, the real-time clock 1206 is implemented using a 32.768 kHz crystal-based clock.

The dispenser 100 may, at least in some embodiments, be programmed by a pharmacist or other user. In such embodiments, the dispenser includes the I/O interface 608. A pharmacist or other authorized user may use I/O interface 608 to program the first and second timers according to the prescription parameters. A pharmacist or other authorized user may also use the I/O interface 608 to set, for example, the date/time, the number of pills disposed in the insert 104, the time between doses, and the prescription length. In some embodiments, the pharmacist or other authorized user may supply additional commands, via the I/O interface 608, such as a reset settings commands (e.g., a clear number of pills and time between doses), a show solenoid status command, a lock solenoid command, an unlock solenoid command, a read battery voltage command, a read firmware version command, and a read hardware version command. These commands may be input, for example, via a command line software run by a computer that is connected to the I/O interface. It will be appreciated that the I/O interface 608 may be implemented using any one of numerous suitable interfaces, so long as the processing system 810 and battery 1204 are properly configured or if a separate device or element (not illustrated) is used to translating the programming signals. As previously noted, in one embodiment the I/O interface 608 is implemented using a suitable USB connector. Thus, the dispenser 100 may be connected to an external computing device via the I/O interface 608. It will additionally be appreciated that the dispenser 100 may be preprogrammed from the factory. Therefore, in at least some embodiments, there is no need for programming by the pharmacist before loading the dispenser 100 with the prescription.

As FIG. 12 also depicts, the dispenser 100 may also, at least in some embodiments, include a non-volatile memory 1208 and/or a tamper microswitch 1205. The non-volatile memory (NVM) 1208, when included, is in operable communication with the processing system 810, and is configured to store operational data indicative of dispenser operation. The operational data that the NVM 1108 stores may vary. Because the processing system 810 itself includes flash memory, the operational data may be stored even without the NVM 1208. However, when flash memory is used, any stored operational data will be lost if the dispenser 100 powers down; thus, it is beneficial to include the NVM 1108.

Memory will also allow the device to keep an activity log, to store device and patient activity, including but not limited to, device activation records, first timing periods without an insert rotation, unreached first timing periods, and how many times the wake button was pressed, for example.

The tamper microswitch 1205, when included, is triggered by a predetermined event. This predetermined event is movement of the lock rod 804 that is not initiated by the solenoid 802 (or motor). The tamper microswitch 1205, when triggered by this event, may send a signal to the processing system 810 that causes the event to be recorded or may cause the processing system 810 to command the insert lock 800 to the lock position or both. The incorporation of the tamper microswitch 1205 allows for a tampering detection system. It will be understood that a microswitch may be added to any of the locking systems of the dispenser 100.

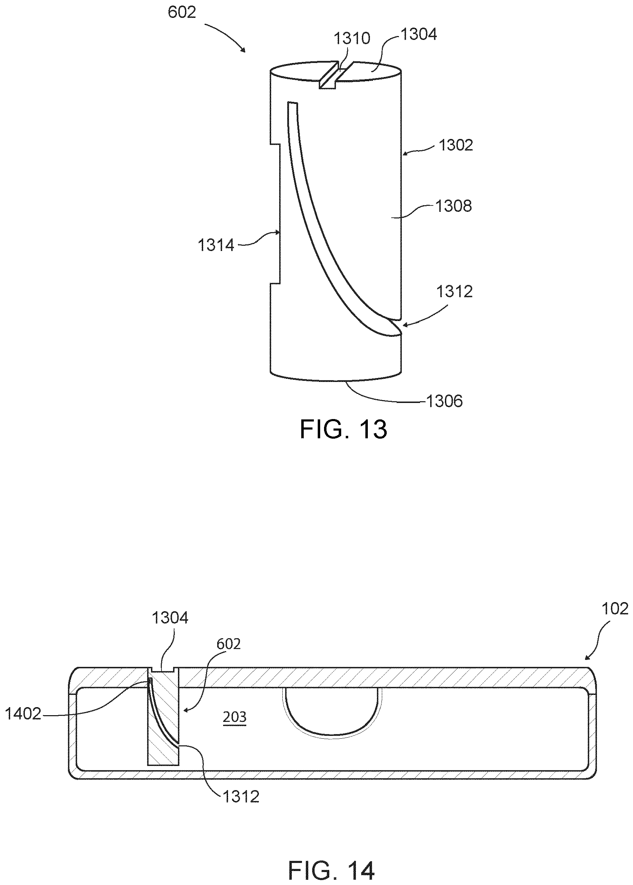

Though not depicted in FIG. 12 or previously described, it will be appreciated that the dispenser 100 may be implemented with various other functions and devices. For example, it may include an alarm function, sensors that enable a tampering circuit, a data collection circuit, or a GPS circuit, just to name a few. The dispenser 100 may also incorporate blockchain as a means to track or generate data. The dispenser 100 may also include wireless connection hardware such as, for example, Wi-Fi or Bluetooth connection hardware. The wireless communication, when included, enables the dispenser 100 to send alerts to a user's phone. A wireless system also allows for wireless programming of the dispenser, and for system data and alerts to be provided to care providers or researchers or to other third parties. A wireless system also reduces the need for memory in the dispenser 100, as the operational data can be wirelessly transmitted to a computer to be stored.
It was noted above that the dispenser 100 may, at least in some embodiments, additionally include an emergency dose capsule 602 and/or an external lock system 610 and/or various types of housing lock systems. Each of these various features and devices will now be described, beginning with the emergency dose capsule 602. In doing so, reference should first be made to FIGS. 13 and 14.

The emergency dose capsule 602, when included, is disposed in the housing inner volume 203 and is accessible via the outer surface 204 of the housing 102. The emergency dose capsule 602 is preferably configured as a child-proof capsule and includes a cylindrical body 1302 having a first end 1304, a second end 1306, and an outer surface 1308. A coin-turn slot 1310 is formed in the second end 1306, and at least one spiral groove 1312 is formed in the outer surface 1308. The emergency dose capsule 602 also includes an opening 1313 that allows access, by a user, to the emergency dose, which is an individual portion of a substance and may be an emergency dose of medication.

As shown in FIG. 14, the emergency dose capsule 602 is disposed of within the inner volume 203 of the housing 102, with the first end 1304 forming part of the outer surface of the housing 102. For each spiral groove 1312, there is a protrusion 1402 formed on a portion of the inner surface 701. Each protrusion 1402 is fitted to the width of the spiral groove 1312 such that as the emergency dose capsule 602 is rotated, the protrusion 1402 lifts the emergency dose capsule 602 according to the pathway of the spiral groove 1312.

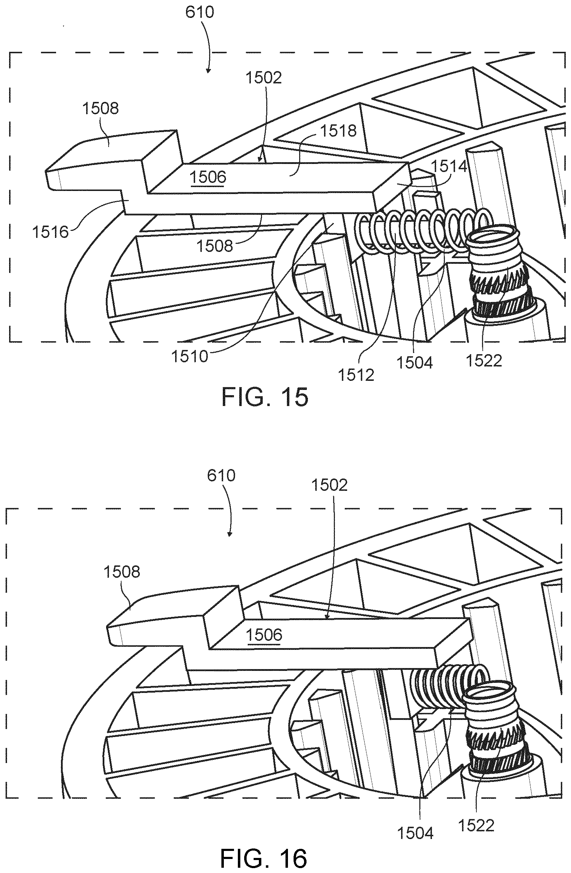

Turning now to FIGS. 15 and 16, one embodiment of the external lock system 610 is depicted. The external lock system 610 is selectively movable between a lock position and an unlock position. In the lock position, which is the position depicted in FIG. 15, the external lock system 610 prevents rotation of the insert 104, and in the unlock position, which is the position depicted in FIG. 16, the external lock system 610 does not prevent rotation of the insert 104. Although the external lock system 610 may be variously configured to implement this functionality, in the depicted embodiment it includes an operator arm 1502 and a lock spring 1504.

The operator arm 1502 is disposed at least partially within the housing 102 and includes a main body 1506, an operator tab 1508, a lock flange 1510, and a spring mount 1512. The main body 1506 is disposed entirely within the housing 102 and has a first end 1513, a second end 1516, an upper surface 1518, and a lower surface 1520. The operator tab 1508 is coupled to, or is formed integrally with, the main body 1506. The operator tab 1508 extends upward from the upper surface 1518 and, at least in the depicted embodiment, extends up through a slot opening 612 formed in the first portion 402 of the housing 102 (see FIG. 6). The operator tab 1508 is sized to allow a user to engage it with sufficient force to overcome the spring force supplied by the spring 1504.

The lock flange 1510 extends perpendicularly from the lower surface 1520 and is sized to fit within the previously described rod catch spaces 512. Thus, as FIG. 16 depicts, when the external lock system 610 is in the lock position, the lock flange 1510 is disposed in one of the rod catch spaces 512. Conversely, when the external lock system 610 is in the unlock position, the lock flange 1510 is not within one of the rod catch spaces 512.

The external lock system 610 is biased toward the lock position via the lock spring 1504. The lock spring 1504 is anchored to a pole 1522 that is mounted within the housing 102 and engages the lock flange 1510. A portion of the lock spring 1504 also surrounds the spring mount 1512, which extends perpendicularly from the lock flange 1510. Thus, as alluded to above, when a user engages the operator tab 1508 and supplies sufficient operator force to overcome the spring force supplied by the spring 1504, the external lock system 610 is moved to the unlock position. If the operator force is removed or is not sufficient to overcome the spring force, the external lock system 610 will return to (or remain in) the lock position. The external lock system 610 thereby prevents an accidental rotation of the insert 104 and may thus be referred to as a child lock.

The housing lock system, when included, may be variously configured, and implemented. It will be appreciated that there is a large variety of suitable housing locking mechanisms available, including, but not limited to screws, bolts, adhesives, clips, latches, mounts (for example bayonet mounts), snap fit joints, and magnets. Further, ti will also be appreciated that the housing lock mechanisms may be used alone or in combination with each other and may incorporate RFID, biomarker, or other identity verification systems.

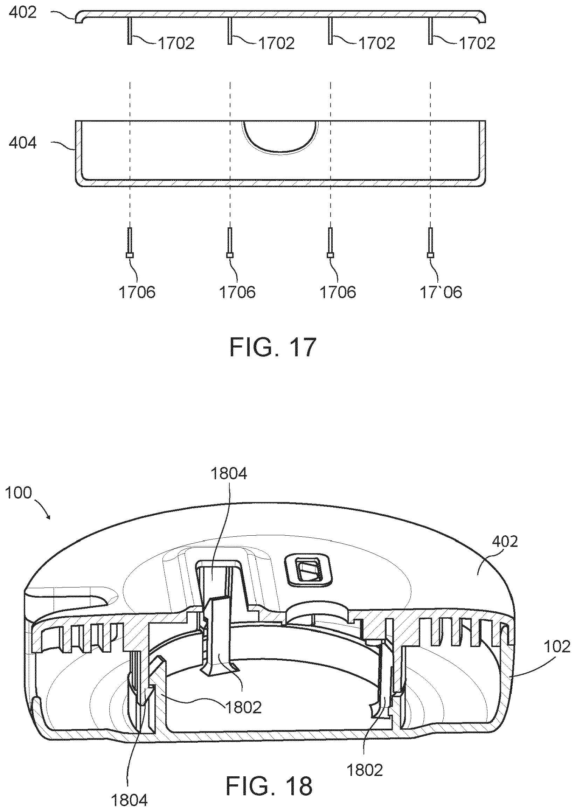

In one embodiment, the housing lock system 1600 is implemented using a tamper-resistant screw system. In such an embodiment, and as FIG. 17 depicts, the first portion 402 of the housing 102 may have a plurality of threaded receptacles 1702 formed thereon, and the second portion 404 of the housing 102, may have a plurality of fastener openings 1704 formed therein. In such a case, a tamper resistant screw 1706 is inserted through each of the fastener openings 1704 and is threaded into a corresponding one of the threaded receptacle 1702. It will be appreciated that in some embodiments the threaded receptacles 1702 are formed on the second portion 404 and the fastener openings 1704 are formed on the first portion 402. It will also be appreciated that regular non-tamper evident screws may be used. A screw-based system allows the dispenser housing to be unlocked and reopened after entering a locked state.

Being able to unlock the housing lock system 1970 may be beneficial if it is foreseeable that there may be instances in which insert rotation is locked, but there is a need to open the housing 102. For example, if the battery 1204 dies, and insert rotation is permanently locked. It also may be beneficial to have an unlockable dispenser in order to refill the insert with a substance, or in the case when the dispenser is intended to be fully controlled by the consumer, for example, the dispenser has been arraigned to dispense a regular dose of aspirin.

In another embodiment, the housing lock system is implemented using a plurality of snap lock features. As is generally known, snap lock features automatically fasten two portions of a device or component together when pushed into position. One example of housing lock system 1800 that is implemented using snap lock features is depicted in FIG. 18. As illustrated therein, the first portion 402 of the housing 102 includes a plurality of first snap lock buckles 1802 (only two visible), and the second portion 404 of the housing 102 includes a plurality of second snap lock buckles 1804 (only three visible). The first and second portions 402, 404 of the housing 102 are locked in place when, as illustrated, each of the first snap lock buckles 1804 locks together a separate one of the second snap lock buckles 1804.

The snap lock system 1800 may serve to keep the housing permanently locked after initiation of the locked state. Thus, when the dispenser 100 is assembled, it is permanently locked. This snap lock system may be useful with disposable embodiments.

It will be appreciated that there are many forms of snap locks that are commonly used and known, such as the embodiment depicted herein. However, various other embodiments may use alternate snap lock styles for the housing lock system 17700.

As noted above, embodiments may include housing lock systems which differ from screw or snap lock-based systems, for example, these deviations may include a padlock system. The padlock may be a keyed padlock or a combination padlock for example. The padlock locking the housing by being operably threaded through the top housing portion and the bottom housing portion and locked.

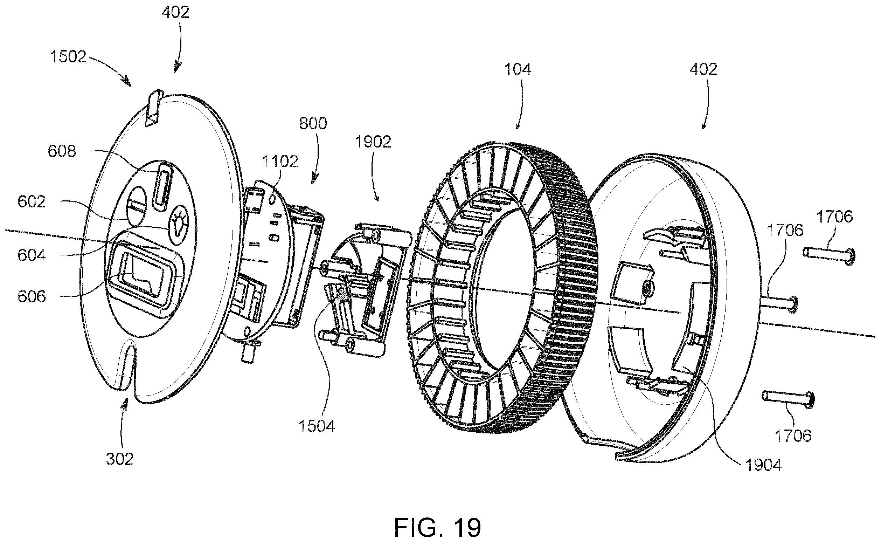

For completeness, an exploded view of one particular preferred embodiment of a dispenser 100, which includes various ones of the features described herein, is depicted in FIG. 19. As depicted, the first portion 402 of the housing 102 includes the opening 302, the emergency dose capsule 602, the wake switch 604, the display device 606, the I/O interface 608, and the operator arm 1502 of the external lock system 610.

In this embodiment, the second portion 404 is attached to the first portion 402 using the previously mentioned tamper resistant screws 1706. The screws 1706 are also used to secure in place, within the inner volume 203, the printed circuit board 1102, which has the processing system 810 and the various components mounted thereon along with the insert lock 800. A support structure 1902, which is also secured in the inner volume via the screws 1706, and which is used to support the printed circuit board 1102, includes the lock spring 1504 and pole 1522 (not visible). When the dispenser 100 is assembled, the insert 104 is kept in alignment via insert supports 1904 formed on the inner surface of the second portion 404, and surrounds the support structure 1902 and the printed circuit board 1102.

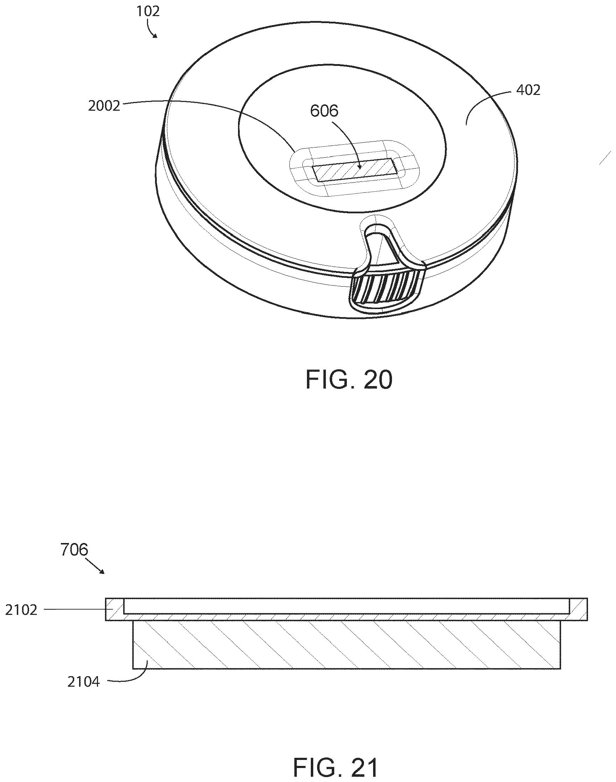

In some exemplary embodiments, the housing 102 and display device 606 may be configured to allow display device 606 to be removed and replaced, as needed or desired. One such embodiment is depicted in FIG. 20. As illustrated therein, a receptacle 2002 is formed in the first portion 402 of the housing 102. Additionally, as depicted in FIG. 21, the display device 606 for this embodiment includes a connector portion 2102, which may be plastic, that is configured to snap or otherwise operably integrate the display device in the receptable 2002. In at least one embodiment the connector portion 2102 locks the display device 606 in the receptacle 2002.

As FIG. 21 also depicts, the connector portion 2102 may include an inner housing portion 2104. The inner housing portion 2104 may contain elements of the dispenser 100, including, for example, the processing system 810, the insert lock 800, or both. This allows these elements to be removed from the dispenser 100 by releasing the connector portion 2102 from the receptacle 2002. Thus, if the dispenser 100 is to be deposed of, the electrical devices may be preserved simply by popping them out of the dispenser 100.

In at least one exemplary method of the present invention, the dispenser of the invention is provided to a user and a substance is provided to the user, the substance operably enclosed by at least one of its compartments. This is achievable when the dispenser has been made to contain a substance. In theory, a user may be provided a dispenser but may not have access to any of the substance within the dispenser. Therefore, in this embodiment, both the dispenser of the present invention is provided, and the substance is provided to the user through the dispenser. In providing a substance to a user through the dispenser, the user has access to the substance according to the parameters of the dispenser.

In at least one exemplary embodiment, the user may provide a user with a substance; and the user may be one and the same. This is to allow a user to prepare his own dispenser or the dispenser of another person. It will also be appreciated that; a user may be provided with a dispenser without a substance within the dispenser.

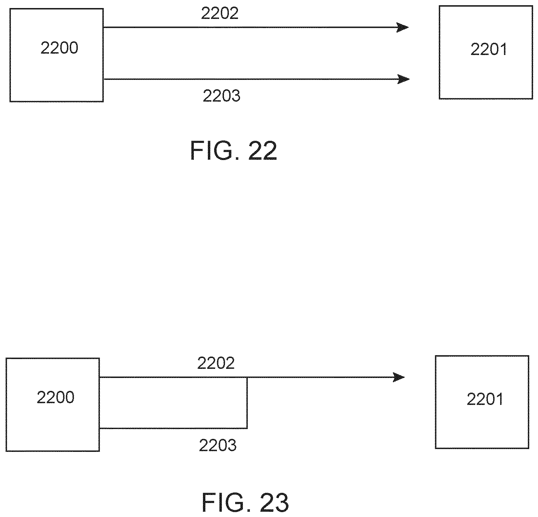

FIG. 22 demonstrates that a provider 2200 may present the user 2201 with both the dispenser 2202 of the present invention and a substance 2203. The arrowed lines of FIG. 22 represent the movement of the dispenser and the user respectively. In at least one exemplary embodiment, as noted above, both the provider and the user may be the same.

FIG. 23 clarifies an exemplary embodiment of the present invention where provider 2200 provides the dispenser 2202 and the substance 2203, with the substance 2203 being provided to the user 2201 while held within the dispenser 2202. This is represented by the dispenser 2202-line and the substance 2203-line combining. This embodiment is an example of a subset of the method captured by FIG. 22.

FIG. 23 demonstrates that a second provider 2204 may provide either the dispenser or the substance to the user 2201 while provider 2200 may provide the opposite of the dispenser or the substance that user 2204 provides. This is to say that the user of the dispenser may receive the dispenser from one party and the substance from another party. The third party 2204 may place the substance into the dispenser before the user receives the device.

A third way to receive the dispenser is for a provider 22700 to provide both the dispenser and the substance to the user, but the user will receive more substance from a third party. For example, a store may provide a dispenser and an initial substance such as dog treats. After completion of the substance received from the initial provider, the user may go to a third-party to refill the dispenser with substance.

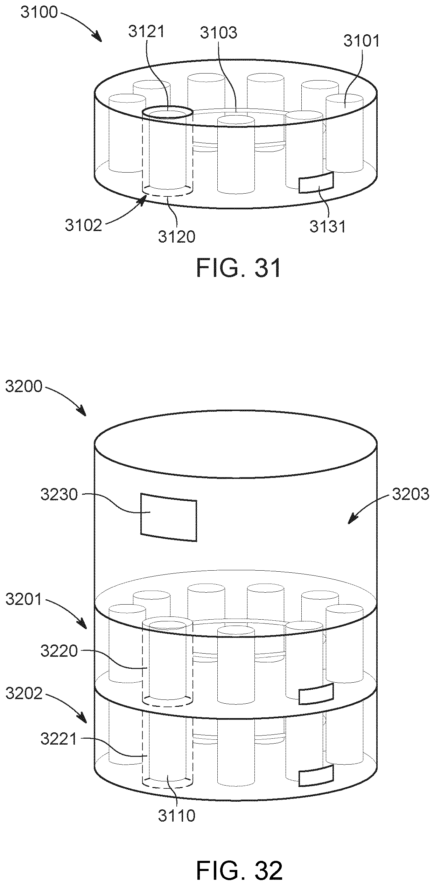

In at least one case where the user receives the dispenser, if the dispenser is disposable, the user may dispose of the dispenser. There are three options for the dispenser if the dispenser is non-disposable: the dispenser may be returned to a provider, the dispenser may be taken to a third party, or the dispenser may be kept indefinitely. Therefore, the dispenser is intended to be kept indefinitely in at least one exemplary embodiment. In at least one exemplary embodiment, the dispenser is intended to be returned, as shown in FIG. 31 , or given to a third party as shown in FIG. 32, for example, a sterilizing company. This sterilizing company may clean the device and return it to the pharmacy.

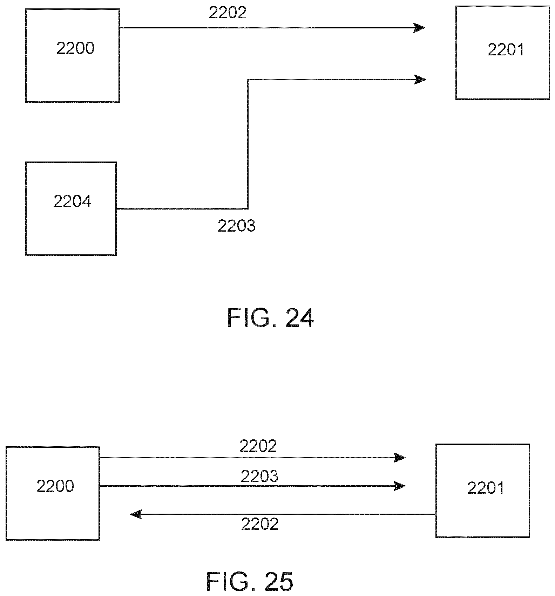

FIG. 25 shows provider 2200 providing a substance 2203 and a dispenser 2202, and the user 2201 providing the dispenser 2202 back to provider 2200.

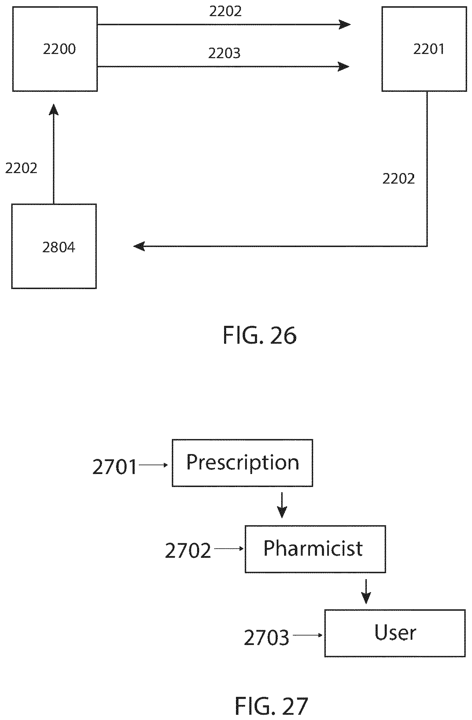

FIG. 26 shows a scenario where the provider 2200 providers a substance 2201 and the dispenser 2202 to the user 2202; the user 2203 returns the dispenser 2201, and in some cases not shown, the dispenser and the substance, to a third party 2204; and the third party 2204 provides the dispenser 2202 back to the original provider 2201.

In some cases, such as in the case with an opioid prescription and a pharmacy, there may be laws which prevent the pharmacy from receiving back opioids which were prescribed to the patient. In such cases, there exist licensed third parties to which the patient may return the opioids. The third parties may handle the opioids and either dispose of the dispenser or return the dispenser to the pharmacy to be reused.

In at least one exemplary embodiment directed to the pharmaceutical industry, a prescription will be presented to a pharmacist. The pharmacist will present a medicine of the prescription to the prescription user within the dispenser, as shown in FIG. 27. In FIG. 27 there are three stages of the process, the prescription stage 2701, the pharmacist stage 2702, and the user stage 2703.

The pharmacist may have a dispenser that is either programmable, non-filled; programmable, filled; not-programmable, filled; or not-programmable, not filled. Programmable refers to the ability of the pharmacist to program the dispenser according to at least one prescription parameter. Programming may involve command line software such that the pharmacist may select the desired parameters and the software then programs the device or the like. Fillable relates to the pharmacist's ability to fill the dispenser with a substance.

A dispenser may come to the pharmacist programmed from a dispenser source so that the pharmacist need not program the dispenser but may just select the dispenser according to its programming. Therefore, there is not necessarily a need for a pharmacist to have a programmable dispenser. A dispenser may also come prefilled from a dispenser source. Therefore, the pharmacist does not always need to fill the dispenser. However, in practice, a pharmacist will likely fill and program a device with at least one prescription parameter.

Just because a device comes to the pharmacist prefilled and preprogrammed does not mean that the device is necessarily unprogrammable or unfillable by the pharmacist. An editable device would allow the pharmacist to alter the settings of the pre-set device and be useful in such cases where the prescription deviates from the prefilled options that a pharmacist has available.

In at least one exemplary embodiment of the present invention, the pharmacist receives a patient prescription, the pharmacist selects a programmable dispenser of the present invention, programs the dispenser according to the prescription parameters, and fills the device according to the prescription. In at least one exemplary embodiment, the pharmacist then gives the device to the patient. This modifies stage 2702 of FIG. 27.

In at least one exemplary embodiment directed to the pharmaceutical industry, the pharmacist receives a patient prescription, the pharmacist may select a preprogrammed dispenser of the present invention, and if the device is not prefilled, the pharmacist may fill the device according to the prescription. This modifies stage 2702 of FIG. 27.

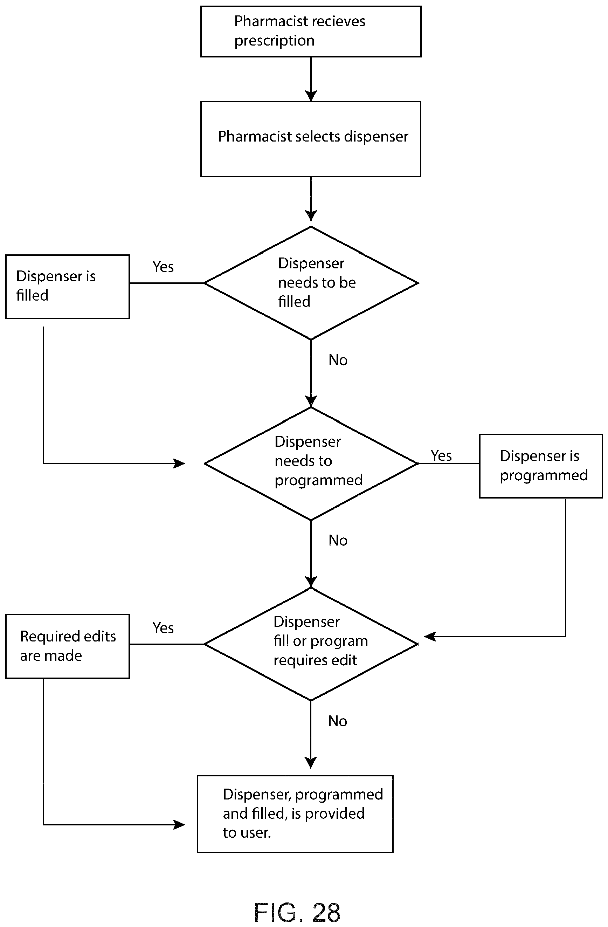

Therefore, it can be seen, as in demonstrated by FIG. 28, that in at least one embodiment of the method, the pharmacist receives the prescription, selects the dispenser, fills the dispenser if needed, programs the dispenser if needed, and edits the programming or fill of the dispenser if needed. The pharmacist then supplies the dispenser and substance to the patient.

In at least one exemplary embodiment, the patient may take the filled and programmed device from the pharmacist, initiate a wake trigger, and take the drug from the first compartment.

In at least one exemplary embodiment, the user of the device may be the one who fills the device with a substance and programs the device according to a desired dispensing protocol.

This user-set device is also useful in the pharmaceutical industry with any drug. The patient may wish to control access to a substance the pharmacist has not provided in a dispenser or that the user himself has received, for instance, an over-the-counter medication. Some medicines, such as ibuprofen, have daily use limits but are sold over the counter in a store. The user may wish to gain such medication, place the medication in a dispenser, program the dispenser, and provide the dispenser to themselves or a user who is then subjected to the added control that the dispenser grants. There are users who would benefit from this method, for example, a person who is forgetful of how much medicine they have already taken.

So therefore, it can be said in at least one exemplary embodiment that the user programs a dispenser according to a dispensing protocol, fills the dispenser according to the desired dispensing protocol, and sets the device to initiate the dispensing protocol.

In at least one exemplary embodiment, the user may buy a preprogrammed and prefilled dispenser, perform a wake event, and use the device according to the programmed dispensing protocol. In at least one exemplary embodiment, the device is only prefilled; in another, the device is only preprogrammed.

In at least one exemplary embodiment of the present invention, the dispenser may have a reset function that resets the first and second timers of the device to the inactive state but allows for them to be reinitiated by a wake event. This allows the dispenser housing to be sold with or without an insert so that the user may buy the first insert or additional inserts.

As demonstrated by FIG. 35, in at least one exemplary embodiment of the present invention the dispenser timers programmed, the dispenser is filled with a substance, the substance kept within the dispenser is transferred to a user, the user triggers the wake event, and the system locks after a predetermined event, the event may be the rotation of the compartment a set distance or the occurrence of set period of time, for example. The locking of the device is followed by an initiation of the next time interval of the first timing system, the first timing system then causes the device to unlock unless the second time has expired.

As demonstrated by FIG. 31, in at least one example embodiment, the dispenser of the present invention is programmed; the substance is inserted in the compartments; the device is transferred to a user; the user triggers a wake event and accesses the drug; the rotation of the device locks; the first timing period is started; the second time period is started; the first timing period expires unlocking the dispenser; and the device remains unlocked until either the second timing system expires or a subsequent wake event occurs. If the subsequent wake event occurs before the second timing system completes, the steps after the subsequent wake event repeat until the second time period completes and locks the device. The subsequent wake event may be, for example, the turning of the insert a set distance such that a micro-switch is triggered. Thus, insert rotation could be locked after a new compartment has been rotated in under the housing opening.

In at least one exemplary embodiment, the dispenser is programmed through a USB port with the date and time of the prescription, the number of substance units (e.g. pills) inserted, and the access frequency. The device is transferred to the user after being loaded with a drug according to a patient's prescription. The patient may press a wake button, and the device may display its current status: device not programmed, requires the pharmacist to program, normal operating display (next dose and pills left), or a low battery screen, or any information, alert, or statement, the device is programmed to display. The first wake button press may be an initial predetermined event that starts the system, or the first rotation of the insert may be the initial predetermined event. The occurrence of this predetermined event moves the solenoid from the lock position to the unlocked position. The patient may then rotate the insert by one compartment. The rotation of the insert triggers a microswitch which causes the solenoid to move the rod back to the locked position and starts the first and second timing systems.

Once the first timing period expires, the device may automatically unlock or be unlocked if the patient presses a wake switch to wake the device after the completion of the first timing period and unlock it. In at least one exemplary embodiment, the device can then update the number of pills left based on the number of compartments left to be rotated.

The device is then locked based on the number of pills left or the end of the second time period, which marks the end of the subscription. By having the patient wake the device to access the pills or providing a set time period between each dose, the user is expected to use fewer pills than the patient normally would in a pill bottle prescription.

The dispenser housing may have additional systems connected to the circuit of the dispenser, including but not limited to RIFD or biometric sensors, for example, a breathalyzer, an optical scanner, or a fingerprint scanner. These systems may be used to verify the identity of a user before either the insert or the housing is unlocked.

The dispenser housing or insert may incorporate a substance denaturant system that is activated after the end of the subscription time period (i.e., the second timing system's period), by the processing system, filling the compartments of the insert with a denaturant to denature any remaining substance. This is useful if the substance is an addictive drug, for example, an opioid.

As discussed above, the timing systems may be preprogrammed during manufacturing, and in such cases the dispenser housing may be marked or color-coded according to its preprogrammed timing systems. For example, a preprogrammed dispenser having a period of one month and a frequency of twice a day may have a blue housing.

In at least one exemplary embodiment of the present invention, a dispenser may have an opening on the top and bottom portion of the dispenser housing, creating a through channel in the dispenser housing. This will allow for the dispensers to be stacked in a base repository and allow multiple dispensers in the base repository to deposit their respective substances at once.

FIG. 31 shows a stackable dispenser 3100 with compartments 3101, opening 3102, and compartment support 3103. Together compartments 3101 and compartment support 3103 make up an insert. In at least one exemplary embodiment, compartments 3101 have an upper opening 3121 as well as a lower opening not shown. It will be appreciated that the shape of compartments 3101 need not be cylindrical as shown. through opening 3102 also has an upper opening 3121 and a lower opening 3120. The dispenser housing keeps substance in the compartments 3101 until the compartments 3201 are rotated to align. A compartment is passed through by substance when the compartment aligns with the slot. The dispenser includes the processing system of the present invention which is housed on a circuit in the inner volume of the dispenser. In at least one exemplary embodiment, the dispenser housing contains a radio frequency tag tag 3131.

The dispenser housing embodiment shown in FIG. 32 is optimal for containment in a base repository. FIG. 38 shows a base repository 3200 with two stacked dispensers: dispenser 3201 and dispenser 3202 stacked inside a base cavity 3203. The dispenser 3201 and dispenser 3202 are stacked so that the opening 3220 of dispenser 3201 is over the opening 3221 of dispenser 3202, and thus the two openings form one opening. This alignment allows for a substance in the upper dispenser(s), in this case dispenser 3202, to pass through compartment of lower dispenser(s), here compartment 3110 of dispenser 3201 and be dispensed. Multiple dispensers may be stacked. The repository may have an upper cover placed on top of the highest dispenser, in this case, dispenser 3202 to prevent substance from entering the cavity 3203 of the base repository 3200. The base repository may have a display screen 3830.

Thus in at least one embodiment, there is a first device comprising an (i) housing having an inner surface, an outer surface and side wall defining a housing inner volume; (ii) a slot formed on the side wall extending between the inner and outer surfaces; (iii) an insert rotationally mounted within the housing inner volume comprising a plurality of compartments along a proximal edge thereof such that only one of the compartments at a time is accessible via the slot; and (iv) an RFID configured to provide information about said first device and the substances contained therein;
a second device comprising a (i) housing having an inner surface, an outer surface and side wall defining a housing inner volume, wherein the perimeter defined by the side wall of the first device and second device are the same; (ii) a slot formed on the side wall extending between the inner and outer surfaces; (iii) an insert rotationally mounted within the housing inner volume comprising a plurality of compartments along a proximal edge thereof such that only one of the compartments at a time is accessible via the slot; and (iv) an RFID configured to provide information about said first device and the substances contained therein;
a base repository configured to receive the first device and second device comprising (i) an enclosure defined by the first device and second device side walls, thereby allowing the slots of the first device and second device to align; (ii) a processing system configured on a printed circuit board operationally disposed in base repository, further comprising sensors to identify the first device and second device, wherein said processing system provides for rotating the insert of the first device relative to the insert of the second device; and (ii) a dispensing area for collecting the substances contained within the aligned compartments of the first device and second device.

There may be any number of the first and second devices stacked unless otherwise limited by the volume of the base repository. A base repository can be made for any dispenser of the present invention.

## Claims

1. A dispenser comprising:
a housing having an inner surface and an outer surface, the inner surface defining a housing inner volume, the housing having an opening formed therein that extends between the inner and outer surfaces;
an insert rotationally mounted within the housing inner volume and rotatable relative to the housing, the insert comprising a plurality of compartments along a proximal edge thereof, the insert and opening disposed and dimensioned such that only one of the compartments at a time is accessible via the opening, and a different one of the compartments is accessible via the opening each time the insert is rotated a predetermined amount;
an insert lock disposed within the housing inner volume and selectively movable between a lock position, in which the insert lock prevents rotation of the insert, and an unlock position, in which the insert lock does not prevent rotation of the insert; and
a processing system disposed within the housing inner volume and in operable communication with the insert lock, the processing system configured to (i) move the insert lock from the lock position to the unlock position at a first time period after a predetermined event, (ii) move the insert lock from the unlock position to the lock position each time the insert is rotated, and (iii) prevent the insert lock from being moved from the lock position to the unlock position after a second predetermined time period,
wherein the first time period is uniform in length or changes in length at least once during the second predetermined time period.

2. The dispenser of claim 1, wherein the first time period increases in length of time by a fixed amount of additional time after the predetermined event.

3. The dispenser of claim 1, further comprising a second insert lock disposed on the outer surface of the housing and selectively movable between a lock position, in which the second insert lock prevents rotation of the insert, and an unlock position, in which the second insert lock does not prevent rotation of the insert.

4. The dispenser of claim 1, further comprising a child-proof emergency dose capsule disposed in the housing inner volume and accessible on the outer surface of the housing.

5. The dispenser of claim 1, further comprising a display screen disposed on the outer surface of the housing operably connected to the processing system.

6. The dispenser of claim 1, wherein the insert is configured to act as a rachet, and a pawl, disposed within the housing volume and operably connected to the insert, is configured to limit the insert to rotation in the first rotational direction.

7. The dispenser of claim 1, wherein the processing system is configured on a circuit comprising a processor, a clock, a controller, a battery, and a memory housed on a printed circuit board operationally disposed in the inner volume of the housing.

8. The dispenser of claim 1, further comprising a housing lock system (i) disposed within the housing volume and accessible on the outer surface of the housing and (ii) selectively configurable between a locked state, in which the interior housing volume is inaccessible to a user, and an unlocked state in which the interior housing volume is accessible to the user.

9. The dispenser of claim 8:
wherein the housing lock system comprises a tamper evident screw system; or
wherein the housing lock system comprises a snap lock system.

10. A method for a timed dispenser comprising:
providing a housing having an inner surface and an outer surface and an opening extending between the inner and outer surfaces;
rotating an insert comprising a plurality of compartments mounted within the housing such that only one of the compartments is accessible via the opening at a time;
actuating an insert lock between a lock position preventing rotation of the insert, and an unlock position; and
programming the insert lock via a processing system configured to transition between the lock position and unlock position at a first time period after a predetermined event, (ii) move the insert lock from the unlock position to the lock position each time the insert is rotate a predetermined amount;
programing the insert lock via a processing system configured to transition between the lock position and unlock position at a second predetermined time period;
dispensing a substance contained in the applicable compartment when both first and second predetermined time period criteria are met, and, optionally,
providing to a user a substance, the substance operably enclosed by at least one of the plurality of medication compartments.

11. The method of providing a dispenser for a patient of claim 10, wherein the first time period increases in length of time by a fixed amount of additional time after the predetermined event.

12. The method of providing a dispenser for a patient of claim 10, further comprising a second insert lock disposed on the outer surface of the housing and selectively movable between a lock position, in which the second insert lock prevents rotation of the insert, and an unlock position, in which the second insert lock does not prevent rotation of the insert.

13. The method of providing a dispenser for a patient of claim 10:
further comprising a child-proof emergency dose capsule disposed in the housing inner volume and accessible on the outer surface of the housing; or
further comprising a display screen disposed on the outer surface of the housing operably connected to the processing system; or
wherein the insert is configured to act as a rachet, and a pawl, disposed within the housing volume and operably connected to the insert, is configured to limit the insert to rotation in a first rotational direction; or
wherein the processing system is configured on a circuit comprising a processor, a clock, a controller, a battery, and a memory housed on a printed circuit board operationally disposed in the inner volume of the housing.

14. The method of providing a dispenser for a patient of claim 10, further comprising a housing lock system (i) disposed within the housing volume and accessible on the outer surface of the housing and (ii) selectively configurable between a locked state, in which the interior housing volume is inaccessible to a user, and an unlocked state in which the interior housing volume is accessible to the user, and, optionally:
wherein the housing lock system comprises a tamper evident screw system; or
wherein the housing lock system comprises a snap lock system.

15. A dispensing system comprising:
a first device comprising a housing having an inner surface, an outer surface and side wall defining a first inner volume; a first opening formed on the side wall extending between the inner and outer surfaces; an insert rotationally mounted within the first inner volume comprising a plurality of compartments such that only one of the compartments at a time is accessible via the first opening; and an RFID configured to provide information about said first device and the substances contained therein;
a second device comprising a housing having an inner surface, an outer surface and side wall defining a second inner volume, wherein the perimeter defined by the side wall of the first device and second device are the same; a second opening formed on the side wall extending between the inner and outer surfaces; an insert rotationally mounted within the second inner volume comprising a plurality of compartments such that only one of the compartments at a time is accessible via the slot; and an RFID configured to provide information about said first device and the substances contained therein; and
a base repository configured to receive the first device and second device comprising an enclosure defined by the first device and second device side walls, thereby allowing the first opening of the first device and the second opening of the second device to align; a processing system disposed in base repository, comprising sensors to identity the first device and second device, wherein said processing system provides for rotating the insert of the first device relative to the insert of the second device; and a dispensing area for collecting the substances contained within the aligned compartments of the first device and second device.
